# EUROPÄISCHE PATENTSCHRIFT

(11) **EP 3 412 643 B1**
(45) Veröffentlichungstag und Bekanntmachung des Hinweises auf die Patenterteilung: **13.11.2019**
(21) Anmeldenummer: 18176765.8
(22) Anmeldetag: 08.06.2018
(51) Int. Cl.: C05G 3/08

(54) **ZUSAMMENSETZUNGEN MIT N-((3(5)-METHYL-1H-PYRAZOL-1-YL)METHYL)ACETAMID UND DE-REN VERWENDUNG ZUR HERSTELLUNG VON LAGERFÄHIGEN FERTIGDÜNGEMITTELN MIT DUALER STICKSTOFFSTABILISIERUNG**
COMPOSITIONS COMPRISING N- ((3 (5) -METHYL-1H-PYRAZOL-1-YL) METHYL) ACETAMIDE AND THEIR USE FOR PREPARING STORAGE STABLE READY FERTILISERS WITH DUAL NITROGEN STABILISATION
COMPOSITIONS CONTENANT N-((3(5)-MÉTHYL-1H-PYRAZOLE-1-YL)MÉTHYL)ACÉTAMIDE ET SON UTILISATION DANS LA PRÉPARATION D'ENGRAIS EN TANT QUE PRODUIT FINI STOCKABLE À DOUBLE STABILISATION DE L'AZOTE

(30) Priorität: 08.06.2017 DE 102017005463
(43) Veröffentlichungstag der Anmeldung: 12.12.2018
(73) Patentinhaber: SKW STICKSTOFFWERKE PIESTERITZ GmbH, 06886 Lutherstadt Wittenberg (DE)
(72) Erfinder: Radics, Ute, 06901 Kemberg (DE); Plötz, Corinna, 06386 Quellendorf (DE); Schuster, Carola, 06886 Lutherstadt Wittenberg (DE); Göhrmann, Bernd, 06749 Bitterfeld-Wolfen (DE); Hucke, André, 06889 Lutherstadt Wittenberg (DE)
(74) Vertreter: Grünecker Patent- und Rechtsanwälte PartG mbB

(56) Entgegenhaltungen:
- EP-A2- 3 072 376
- EP-B1- 2 456 737
- DE-B3-102013 022 031

## Beschreibung

Die vorliegende Erfindung betrifft Zusammensetzungen, enthaltend N-((3(5)-Methyl-1H-pyrazol-1-yl)methyl)acetamid als Nitrifikationsinhibitor, N-(2-Nitrophenyl)phosphorsäure-triamid als Ureaseinhibitor, Pflanzenöl, mindestens ein Wachs und optional ein Verdünnungsmittel, die Verwendung der Zusammensetzung zur Herstellung von lagerfähigen Fertigdüngemitteln mit dualer Stickstoffstabilisierung, ein Verfahren zur Herstellung von lagerfähigen Fertigdüngemitteln mit dualer Stickstoffstabilisierung sowie ein lagerfähiges Fertigdüngemittel.

### Stand der Technik

Weltweit erfolgt die Düngung mit Stickstoff überwiegend mit Hilfe von Harnstoff oder auch Harnstoffmischungen mit anderen Düngerkomponenten wie beispielsweise Ammoniumsulfat.

Die Düngewirkung des Harnstoffs im Ackerboden beruht auf der hydrolytischen Umwandlung vom Harnstoff durch das ubiquitär im Boden vorhandene Enzym Urease zu Ammoniumionen und Hydrogencarbonat. Im biologisch aktiven Boden kann das Ammoniumion unter Einwirkung von Nitrosomas- und Nitrobacter-Bakterien über Nitrit sehr schnell zum Nitrat oxidiert werden.

Durch Nitrifikationsinhibitoren wird die mikrobielle Umwandlung von Ammoniumstickstoff zu Nitratstickstoff im Boden über einen gewissen Zeitraum gehemmt und dadurch eine verbesserte Stickstoffausnutzung durch die Pflanze bewirkt, da das Risiko der Nitratverlagerung deutlich gemindert ist. Gleichzeitig können Lachgasemissionen um über 50 % reduziert werden.

Die Anwendung von mit Nitrifikationsinhibitoren kombinierten Stickstoffdüngern ermöglicht auf Grund ihrer Wirkungsweise sowohl wirtschaftliche als auch ökologische Vorteilseffekte. Infolge der über einen Zeitraum von 4 bis 12 Wochen andauernden Hemmung der mikrobiellen Oxidation des gedüngten Ammoniumstickstoffs zu Nitrat liegt der Stickstoff vorwiegend in Form von Ammonium (für die Pflanzen verfügbar) vor und es werden N-Verluste durch Nitratauswaschung und Lachgasemissionen reduziert. Die Verminderung des Verlustpotenzials erlaubt, verbunden mit gewissen Vorteilen einer ammoniumbetonten Pflanzenernährung, eine Reduzierung des N-Aufwandes bei gleichbleibend hohem Ertragsniveau sowie eine Zusammenlegung von Arbeitsgängen, was zu arbeitswirtschaftlichen Vorteilen führt.

Nachweislich wirkt sich der Einsatz von mit Nitrifikationsinhibitoren versehenen Stickstoffdüngemitteln besonders vorteilhaft in Wasserschutzzonen aus, da durch die verzögerte Nitratbildung der Eintrag dieser N-Form in Gewässer erheblich reduziert werden kann.

Harnstoff ist ein ursprünglich biogenes Stoffwechselprodukt, das durch das Enzym Urease in Ammoniak und Kohlendioxid gespalten wird. Die Reaktion verläuft außerordentlich schnell und effektiv und ist somit für N-Verluste bei der Anwendung von harnstoffbasierten Düngemitteln verantwortlich. Diese sind besonders hoch, wenn der Boden nicht über eine ausreichende Sorptionskraft verfügt, um das frei gewordene Ammoniak in Form von Ammoniumionen zu binden. Dadurch gehen der Landwirtschaft jährlich beträchtliche Mengen an Stickstoff verloren, die auf diese Weise zur Umweltbelastung beitragen und andererseits einen erhöhten Düngemittelbedarf erfordern.

Der Einsatz von Ureaseinhibitoren ist eine effektive Möglichkeit, die unter Normalbedingungen außerordentlich schnell verlaufende enzymatische Harnstoff-Hydrolyse deutlich zu verlangsamen. Durch die Verzögerung dieser Enzymreaktion kann der Düngeharnstoff unzersetzt in tiefere Bodenschichten penetrieren. Außerdem wird das langsam und kontinuierlich entstehende Ammonium vollständiger an den Ton-Humus-Komplex gebunden.

Sich deutlich verändernde Witterungsbedingungen sowie politische Forderungen zur Verbesserung der N-Effizienz geben Anlass, Nitrifikations- und Ureaseinhibitoren bei der N-Stabilisierung von Harnstoff zu kombinieren. Damit werden die bekannten Vorteile des Einsatzes von Nitrifikationsinhibitoren, darunter die Ammonium-betonte Ernährung, die Minderung von Auswaschungs- und Denitrifikationsverlusten, mit einer deutlichen Minderung von Ammoniakverlusten nach der Harnstoffapplikation verbunden. Auf diese Weise können alle wesentlichen N-Verlustpfade signifikant gemindert werden.

Mit dem Einsatz von Harnstoff-Düngern mit dualer Stabilisierung kann zur Reduzierung der Stickstoffüberschüsse in der Landwirtschaft beigetragen werden. Dies ist eine umweltpolitische Forderung, der die landwirtschaftliche Produktion zunehmend gerecht werden muss.

Aus der Literatur ist bekannt, dass bestimmte organische, aber auch anorganische Verbindungen die ureasekatalysierte Harnstoff-Hydrolyse zu hemmen vermögen (vgl. S. Kiss, M. Simihäian, Improving Efficiency of Urea Fertilizers by Inhibition of Soil Urease Activity, Kluwer Academic Publishers (2002)).

Mit der Entdeckung der Phosphorsäureesterdiamide (DD 122 177) sind Verbindungen gefunden worden, die äußerst effektive Ureaseinhibitoren darstellen. Ähnlich wirksam ist eine Reihe von Derivaten des Phosphorsäuretriamids einschließlich des Grundkörpers (vgl. bspw. US 4,540,428, US 4,676,822, US 4,696,693, US 4,537,614, US 4,517,004, EP 0 119 487), von denen das N-(n-Butyl)thiophosphorsäuretriamid (NBPT) (Koch Fertilizer, Produktbezeichnung Agrotain®) und die Kombination aus N-(n-Butyl)thiophosphorsäuretriamid (NBPT) und N-(n-Propyl)thiophosphorsäuretriamid (NPT) (BASF, Produktbezeichnung Limus®) kommerzialisiert wurden.

Als wirksame Nitrifikationsinhibitoren sind eine große Zahl verschiedener Substanzen und Substanzgemische vorgeschlagen worden (siehe u. a. M.E. TRENKEL, Slow- and Controlleded-Release and Stabilized Fertilizers - An Option for Enhancing Nutrient Use Efficiency in Agriculture; International Fertilizer Industry Assoziation (ifa), Paris Oct. 2010).

Für den praktischen Einsatz als Nitrifikationsinhibitoren eignen sich jedoch aufgrund ihrer chemischen und physikalischen Eigenschaften nur wenige der vorgeschlagenen Substanzen.

So ist Dicyandiamid (DCD) einer der seltenen Wirkstoffe, die ohne Zersetzung in eine Harnstoffschmelze eingebracht werden können (vgl. z. B. EP 0 908 430 B1). Zur Gewährleistung einer sicheren nitrifikationsinhibierenden Wirkung müssen jedoch 3 bis 10 Gew.-% Dicyandiamid in Harnstoff eingearbeitet werden, was einen vergleichsweise hohen Wirkstoffanteil bedeutet.

In DE 44 05 392 C1 werden Wirkstoffkombinationen zur Hemmung bzw. Regelung der Nitrifikation beschrieben, mit denen es gelingt, den mit Dicyandiamid als Einzelsubstanz erforderlichen Wirkstoffgehalt um bis zu 80 % zu senken. Ein erfolgreich in der Industrie umgesetztes Beispiel ist die synergistisch wirkende Kombination von Dicyandiamid mit 1H-1,2,4-Triazol.

Die in DE 103 42 551 offenbarte Herstellung eines Feststoffdüngers aus Harnstoff oder Harnstoff/Ammoniumsulfat mit einem Nitrifikationsinhibitorgemisch aus Dicyandiamid und 1,2,4,-Triazol im Gewichtsverhältnis 10:1 ist ein technologisch anspruchsvoller Prozess und setzt sowohl das Vorhandensein der erforderlichen Anlagen als auch des entsprechenden Know-hows voraus. Das Dicyandiamid wird dabei in der Schmelze mit dem Harnstoff granuliert und das 1,2,4-Triazol abschließend als konzentrierte wässrige Lösung auf die Granalienoberfläche aufgetragen. Die enthaltene Wirkstoffkonzentration von 2,0 % bezogen auf den reduzierten Stickstoffgehalt ist jedoch noch relativ hoch.

Zunehmend wird in der Literatur über N-Verlustminderungen bei gleichzeitigem Einsatz von Nitrifikations- und Ureaseinhibitoren berichtet (vgl. M. I. Khali Physical and chemical manipulation of urea fertilzer to limit the emission of reactive nitrogen species; ACS symposium Series (2011) 1072 (Understanding Greenhouse Gas Emissions from Agricultural Management) 149-164) Agriculture, Ecosystems and Environment 221 (2016) 214-221 (Improving fertilizer management in the U.S. and Canada for N₂O mitigation: Understanding potential positive and negative side-effects on corn yields; Diego Abalosa, Simon Jefferyb, Craig F. Druryc, Claudia Wagner-Riddlea)

Harnstoffdüngemittel mit einer Kombination von beiden Arten der N-Stabilisation werden zwar in vielen Patentanmeldungen mit beansprucht wie beispielsweise in WO 2016 137 815 A1 oder CN 103896686 A 2010702, die Herstellung eines wohldefinierten Fertigproduktes mit dualer Stickstoffinhibierung im technischen Maßstab ist bisher jedoch nur mit den Ausnahmeprodukten UFLEXX und UMAXX für den Golf- und Rasen- und Zierpflanzenbereich bekannt geworden.

Für die Anwendung in Feldfrüchten steht ein entsprechendes Fertigprodukt bisher nicht zur Verfügung. An die Herstellung von Spezialdüngemitteln für dieses Marktsegment werden besonders hohe Anforderungen gestellt. Der Nitrifikations- und der Ureaseinhibitor müssen sowohl zusammen als auch im Gemisch zusammen mit Harnstoff über eine lange Lagerzeit verlustfrei kombinierbar sein und dürfen währenddessen miteinander keine Unverträglichkeiten aufweisen. Zusammensetzungen, die zur Herstellung solch eines Kombinationsproduktes eingesetzt werden, müssen die guten Lagereigenschaften von granuliertem Harnstoff erhalten und gleichzeitig die Lagerstabilität der Wirkstoffe garantieren.

Eine alternative Herstellungsvariante für den Einsatz von stickstoffstabilisierten Düngemitteln ist eine Vorortbehandlung von Düngergranulaten mit einer Inhibitormischung. Aus WO 2013 121384 A2 sind Mischungen zur Reduzierung von Lachgas- und/oder Ammoniak-Emissionen mit synergistischen Effekten bekannt. Auch in WO 2016 183511 A1 und WO 2015 168663 A1 werden Zusammensetzungen für eine Vorortbehandlung von Düngergranulaten mit Nitrifikations- und Ureaseinhibitoren beschrieben.

In WO 2016 207210 A1 wird eine Zusammensetzung mit Nitrifikations- und Ureaseinhibitoren beansprucht, allerdings handelt es sich dabei um eine Mischung für eine Vorortbehandlung von Düngergranulaten.

Produkte, die durch eine Vorortbehandlung von Düngergranulaten mit Wirkstoffmischungen hergestellt werden, zeichnen sich in der Regel durch eine begrenzte Lagerfähigkeit aus. Ihr Einsatz ist mit einer Abhängigkeit von freien Mischkapazitäten bei Lohnherstellern und von der Verfügbarkeit der Wirkstoffmischungen verbunden. Die Kontrolle der Einhaltung von Parametern, die nach Düngemittelrecht vorgegeben sind, ist bei Vorortbehandlung deutlich erschwert bzw. unmöglich. Diese Nachteile können durch den Einsatz eines lagerstabilen Fertigproduktes mit konstanter Qualität behoben werden.

In EP 2 456 737 B1 wird die technische Herstellung (350 t) eines harnstoffbasierten Düngemittels mit einem Phosphorsäureamidderivat 2NPT = *N*-(2-Nitrophenyl)phosphorsäuretriamid als Ureaseinhibitor beschrieben, die zu einem langzeitlagerfähigen (ca. 1 Jahr) Fertigprodukt führt.

Als zur Formulierung besonders geeignet wird ein Formulierungsmittel beansprucht, welches neben Wachsen aus n- und Isoparaffinen auch pflanzliche Triglyceride enthält. In einer bevorzugten Ausführungsform der Erfindung besteht das Formulierungsmittel aus 20-40 Gew.-% n-Paraffinen, 40-60 Gew.-% Isoparaffinen und 5-30 Gew.-% pflanzlichen Triglyceriden. Es wird auch beansprucht, dass diesem Formulierungsmittel Pyrazolderivate als Nitrifkationsinhibitoren beigemischt werden könnten.

In EP 1 663 997 B1 werden N-(1H-Azolyl-methyl)amide als Einkomponentenwirkstoffe mit lipophilem Charakter offenbart, die auch bei geringen Aufwandmengen ausgezeichnete nitrifikationshemmende Eigenschaften zeigen und auch in Gegenwart von harnstoffhaltigen Düngemitteln hydrolysestabil sind ohne dass sie vorher in ihre Salze überführt werden müssen, um Wirkstoffverluste infolge Flüchtigkeit zu verhindern. Dies ist umso bemerkenswerter als bekannt ist, dass Harnstoff stets mit geringen Anteilen von freiem Ammoniak begleitet ist, der die sonst schwächer basischen Azolderivate aus ihrem für die Wirkung unerlässlichen Verbund mit Harnstoff verdrängt und somit zu unterkritischen Konzentrationen an Nitrifikationsinhibitor führt, die eine ausreichende Nitrifikationshemmung nicht gewährleistet.

Die DE 10 2013 022 031 beschreibt N-((3(5)-Methyl-1H-pyrazol-1-yl)methyl)acetamid (MPA) als Nitrifkationsinhibitor, der sowohl hydrolyse- als auch in Kombination mit Harnstoff lagerstabil ist.

EP 3072376 offenbart einen flüssigen Düngemittelzusatzstoff, enthaltend einen Ureaseinhibitor (z.B. N-((3(5)-Methyl-1 H-pyrazol-1-yl)methyl)acetamid) und/oder einen Nitrifikationsinhibitor (z.B. 2-NPT).

### Gegenstand der Erfindung

Der vorliegenden Erfindung lag daher die Aufgabe zugrunde, eine Zusammensetzung zur Verfügung zu stellen, die zur Herstellung eines lagerfähigen Fertigdüngemittels mit dualer Stickstoffstabilisierung verwendet werden kann.

Zusammensetzungen, die zur Herstellung solch eines Kombinationsproduktes eingesetzt werden, müssen die guten Lagereigenschaften von granuliertem Harnstoff erhalten und gleichzeitig die Lagerstabilität der Wirkstoffe garantieren.

### Beschreibung der Erfindung

Die Aufgabe, eine Zusammensetzung zur Verfügung zu stellen, die zur Herstellung eines lagerfähigen Fertigdüngemittels mit dualer Stickstoffstabilisierung verwendet werden kann, wird erfindungsgemäß durch die Bereitstellung einer Zusammensetzung gelöst, die N-((3(5)-Methyl-1H-pyrazol-1-yl)methyl)acetamid (MPA) (I) als Nitrifikationsinhibitor, N-(2-Nitrophenyl)phosphorsäuretriamid (2NPT) (II) als Ureaseinhibitor, Pflanzenöl,mindestens ein Wachs und optional ein Verdünnungsmittel, enthält.

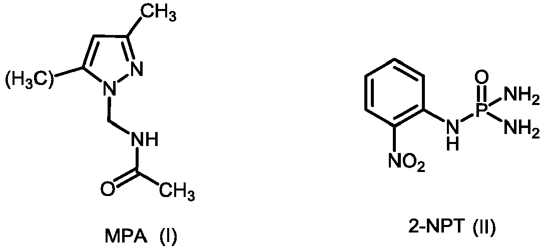

Die Aufgabe wird außerdem durch die Verwendung der erfindungsgemäßen Zusammensetzung zur Herstellung von lagerfähigen Fertigdüngemitteln mit dualer Stickstoffstabilisierung gelöst.

Ferner wird die Aufgabe durch ein Verfahren zur Herstellung von lagerfähigen Fertigdüngemitteln mit dualer Stickstoffstabilisierung gelöst, wobei granulierter Harnstoff mit der erfindungsgemäßen Zusammensetzung behandelt wird.

Außerdem wird die Aufgabe gelöst durch ein lagerfähiges Fertigdüngemittel, enthaltend die erfindungsgemäße Zusammensetzung.

Es hat sich nämlich überraschenderweise gezeigt, dass beim kombinierten Einsatz von 2-NPT mit MPA in einem Harnstofffertigdüngemitel der Wachsanteil im Formulierungsmittel wesentlich geringer sein kann als dies bei der alleinigen Anwendung von Phosphorsäureamidderivaten als Ureaseinhibitoren der Fall ist. Der Wachsanteil konnte auf weniger als ein Zehntel der in EP 1 663 997 B1 beschriebenen Menge reduziert werden, ohne dass sich dabei die anwendungstechnischen Eigenschaften des Fertigproduktes verschlechtern. Dies war nicht vorauszusehen.

Die erfindungsgemäße Zusammensetzung reduziert Nitratverluste um 35 bis 50 Gew.-%. Ammoniakverluste werden nahezu vollständig reduziert, Lachgasemissionen um mehr als 50 Gew.-%.

Die erfindungsgemäße Zusammensetzung enthält neben N-((3(5)-Methyl-1H-pyrazol-1-yl)methyl)acetamid (MPA) (I) als Nitrifikationsinhibitor und N-(2-Nitrophenyl)-phosphorsäuretriamid (2NPT) (II) als Ureaseinhibitor außerdem ein Pflanzenöl und mindestens ein Wachs.

Die Wirkstoffe N-((3(5)-Methyl-1H-pyrazol-1-yl)methyl)acetamid und N-(2-Nitrophenyl)phosphorsäuretriamid sind in einer bevorzugten Ausführungsform in Kombination mit einer der oben oder unten genannten Ausführungsformen in der Zusammensetzung in einer Gesamtmenge von 10-40 Gew.-% enthalten, besonders bevorzugt in einer Gesamtmenge von 15-40 Gew.-%, insbesondere 17-40 Gew.-%, bezogen auf die Gesamtzusammensetzung.

In einer weiteren bevorzugten Ausführungsform in Kombination mit einer der oben oder unten genannten Ausführungsformen enthält die erfindungsgemäße Zusammensetzung 5-30 Gew.-% N-((3(5)-Methyl-1H-pyrazol-1-yl)methyl)acetamid und 5-34 Gew.-% N-(2-Nitrophenyl)phosphorsäuretriamid, besonders bevorzugt 9-28 Gew.% N-((3(5)-Methyl-1H-pyrazol-1-yl)methyl)acetamid und 10-34 Gew-% N-(2-Nitrophenyl)phosphorsäuretriamid, bezogen auf die Gesamtzusammensetzung.

Durch die Verwendung eines Pflanzenöls als Formulierungsmittelbasis muss die Zusammensetzung bei der Herstellung des lagerfähigen Fertigdüngemittels mit dualer Stickstoffstabilisierung nicht erwärmt werden, um fließfähig zu sein. Dies garantiert schonende Bedingungen für die Wirkstoffe und vereinfacht das Herstellungsverfahren für das Düngemittel erheblich.

Unter "dualer Stickstoffstabilisierung" wird erfindungsgemäß die Stabilisierung gegenüber Nitrifikation (mikrobielle Oxidation von Ammoniak bzw. Ammoniumionen zu Nitrat) und gegenüber Abbau durch Urease verstanden.

Als Pflanzenöl eignen sich üblicherweise verwendete Pflanzenöle, wie z. B. Rapsöl, Sonnenblumenöl, Sojaöl, Leinöl, Olivenöl oder Drachenkopföl oder Gemische aus mehreren dieser Öle. In einer bevorzugten Ausführungsform in Kombination mit einer der oben oder unten genannten Ausführungsformen enthält die erfindungsgemäße Zusammensetzung Rapsöl oder Sojaöl.

In einer weiteren bevorzugten Ausführungsform in Kombination mit einer der oben oder unten genannten Ausführungsformen enthält die erfindungsgemäße Zusammensetzung das Pflanzenöl in einer Menge von 30-85 Gew.-%, besonders bevorzugt in einer Menge von 50-80 Gew.-%, insbesondere 60-80 Gew.-%, bezogen auf die Gesamtzusammensetzung.

Als Wachse können polare oder unpolare Wachse natürlichen oder synthetischen Ursprungs wie Polyethylen-, Polypropylen-, Zuckerohr-, Amid-, Bienen- oder Paraffinwachse oder auch Hybridwachse eingesetzt werden. In einer bevorzugten Ausführungsform in Kombination mit einer der oben oder unten genannten Ausführungsformen enthält die erfindungsgemäße Zusammensetzung Paraffinwachs, Polyethylenwachs oder E-rucamidwachs.

In einer weiteren bevorzugten Ausführunsgform in Kombination mit einer der oben oder unten genannten Ausführungsformen enthält die erfindungsgemäße Zusammensetzung das Wachs in einer Menge von 0,1 bis 5 Gew.-%, besonders bevorzugt in einer Menge von 1-4 Gew.-%, bezogen auf die Gesamtzusammensetzung.

Außerdem muss das verwendete Wachs nicht zwingend ein Paraffinwachs sein. Trotz des geringen Wachsanteils bleiben die guten Lagereigenschaften des granulierten Harnstoffs erhalten.

Die erfindungsgemäße Zusammensetzung kann optional ein Verdünnugsmittel enthalten. Das Verdünnungsmittel kann in einer bevorzugten Ausführungsform in Kombination mit einer der oben oder unten genannten Ausführungsformen ausgewählt sein aus einem N-substituierten Lactam, wie N-Methylpyrrolidon, einem Ester, wie Fettsäurealkylester, z. B. Rapsmethylester, Sonnenblumenölmethylester, Sojaölmethylester oder Palmölmethylester, veresterter Abietinsäure sowie den grünen Lösungsmitteln Isopropylidenglycerin und Glycerinformal oder Gemischen daraus.

In einer weiteren bevorzugten Ausführungsform in Kombination mit einer der oben oder unten genannten Ausführungsformen enthält die erfindungsgemäße Zusammensetzung das Verdünnungsmittel in einer Menge von 0 bis 10 Gew.-%, besonders bevorzugt in einer Menge von 4-9 Gew.-%, insbesondere 5-9 Gew.-%, bezogen auf die Gesamtzusammensetzung.

In den erfindungsgemäßen Düngemittelzusammensetzungen weisen nicht nur die Wirkstoffe eine gute Lagerstabilität auf. Auch das harnstoffbasierte Düngemittel selbst bleibt unter praxisrelevanten klimatischen Bedingungen gut lagerfähig.

Zur Formulierung können in einer weiteren bevorzugten Ausführungsform in Kombination mit einer der oben oder unten genannten Ausführungsformen noch weitere, übliche, dem Fachmann bekannte, nichttoxische und physiologisch geeignete Hilfs- und Trägerstoffe, Farbstoffe, Emulgier- oder Dispergiermittel oder Streckmittel (ggf. jeweils in Kombination) eingesetzt werden.

Die erfindungsgemäßen Zusammensetzungen eignen sich zur Herstellung eines lagerfähigen Fertigdüngemittels mit dualer Stickstoffstabilisierung, das N-((3(5)-Methyl-1H-pyrazol-1-yl)methyl)acetamid (MPA) (I) als Nitrifikationsinhibitor und N-(2-Nitrophenyl)phosphor-säuretriamid (2NPT) (II) als Ureaseinhibitor enthält.

Zu diesem Zweck kann in einer bevorzugten Ausführungsform in Kombination mit einer der oben oder unten genannten Ausführungsformen die erfindungsgemäße Zusammensetzung in einer Menge von zum Beispiel 0,05-1 Gew.-%, bezogen auf die Gesamtmenge des Düngemittels, mittels einer Sprüheinrichtung auf granulierten Harnstoff aufgesprüht werden. In einer weiteren bevorzugten Ausführungsform enthält das erfindungsgemäße lagerfähige Fertigdüngemittel 0,1 bis 0,60 Gew.-% der erfindungsgemäßen Zusammensetzung.

Die vorliegende Erfindung soll nun anhand der folgenden Beispiele erläutert werden.

### Beispiele

### Beispiel 1: Zusammensetzung mit 9 % MPA und 10 % 2-NPT

30 g Paraffinwachs werden in 780 g Sojaöl erwärmt, bis eine homogene Mischung entsteht. Man lässt abkühlen und suspendiert mit einem laborüblichen Ultraturrax 90 g MPA und 100 g 2-NPT ein.

### Beispiel 2: Zusammensetzung mit 20 % MPA und 10 % 2-NPT

20 g Polyethylenwachs werden in 610 g Rapsöl erwärmt, bis eine homogene Mischung entsteht. Man lässt abkühlen, verdünnt mit 70 g Rapsmethylester und suspendiert mit einem laborüblichen Ultraturrax 200 g MPA und 100 g 2-NPT ein.

### Beispiel 3: Zusammensetzung mit 15 % MPA und 15 % 2-NPT

10 g Eurucamidwachs werden in 620 g Rapsöl erwärmt, bis eine homogene Mischung entsteht. Man lässt abkühlen, verdünnt mit 70 g Isopropylidenglycerin und suspendiert mit einem laborüblichen Ultraturrax 150 g MPA und 150 g 2-NPT ein.

### Beispiel 4: Wirkstoffstabilität auf Harnstoff

Zur Untersuchung der Wirkstoffstabilität nach Aufbringen der erfindungsgemäßen Zusammensetzung auf Harnstoff wurden 5 kg Harnstoffgranulat mit 20 bis 42 g der erfindungsgemäßen Zusammensetzung imprägniert. Die Proben wurden im verschlossenen Behälter bei Raumtemperatur gelagert und der Wirkstoffgehalt mittels HPLC überprüft.

| Zusammensetzung nach Beispiel | MPA und 2-NPT-WFR¹ in % | Lagerzeit |
|---|---|---|
| (1) | 95 | 9 Monate |
| (2) | 96 | 1 Jahr |
| (3) | 94 | 1 Jahr |

| | | |
|---|---|---|
| ¹ Wiederfindungsrate von MPA und 2-NPT auf Harnstoff nach Lagerung bei Raumtemperatur | | |

## Patentansprüche

1. Zusammensetzung, die N-((3(5)-Methyl-1 H-pyrazol-1-yl)methyl)acetamid (MPA) (I) als Nitrifikationsinhibitor, N-(2-Nitrophenyl)phosphorsäuretriamid (2NPT) (II) als Ureaseinhibitor, Pflanzenöl, mindestens ein Wachs und optional ein Verdünnungsmittel enthält

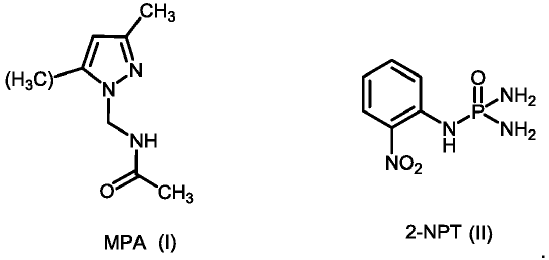

2. Zusammensetzung nach Anspruch 1, **dadurch gekennzeichnet, dass** N-((3(5)-Methyl-1H-pyrazol-1-yl)methyl)acetamid und N-(2-Nitrophenyl)phosphorsäuretriamid (2NPT) in einer Konzentration von 10-40 Gew.-%, bezogen auf die Gesamtzusammensetzung, enthalten sind.

3. Zusammensetzung nach Anspruch 1 oder Anspruch 2, **dadurch gekennzeichnet, dass** das Pflanzenöl in einer Konzentration von 30-85 Gew.-%, bezogen auf die Gesamtzusammensetzung, enthalten ist.

4. Zusammensetzung nach einem der Ansprüche 1-3, **dadurch gekennzeichnet, dass** ein Wachs in einer Menge von 0,1 bis 5 Gew.-%, bezogen auf die Gesamtzusammensetzung, enthalten ist

5. Zusammensetzung nach einem der Ansprüche 1-4, **dadurch gekennzeichnet, dass** ein Verdünnungsmittel in einer Konzentration von 0,1 bis 10 Gew.-%, bezogen auf die Gesamtzusammensetzung, enthalten ist.

6. Zusammensetzung nach einem der Ansprüche 1-5, **dadurch gekennzeichnet, dass** das Verdünnungsmittel ausgewählt ist aus einem N-substituierten Lactam, einem Ester, veresterter Abietinsäure, Isopropylidenglycerin, Glycerinformal oder Gemischen daraus.

7. Zusammensetzung nach Anspruch 6, **dadurch gekennzeichnet, dass** das N-substituierte Lactam ausgewählt ist aus N-Methylpyrrolidon und/oder der Ester ausgewählt ist aus einem Fettsäurealkylester.

8. Verwendung der Zusammensetzung nach einem der Ansprüche 1-7 zur Herstellung von lagerfähigen Fertigdüngemitteln mit dualer Stickstoffstabilisierung.

9. Verfahren zur Herstellung von lagerfähigen Fertigdüngemitteln mit dualer Stickstoffstabilisierung, **dadurch gekennzeichnet, dass** man granulierten Harnstoff mit der Zusammensetzung nach einem der Ansprüche 1-7 behandelt.

10. Lagerfähiges Fertigdüngemittel mit dualer Stickstoffstabilisierung, enthaltend die Zusammensetzung nach einerm der Ansprüche 1-7.

11. Lagerfähiges Fertigdüngemittel nach Anspruch 10, **dadurch gekennzeichnet, dass** das Fertigdüngemittel 0,1 bis 0,60 Gew.-% der Zusammensetzung nach einem der Ansprüche 1-7 enthält.

## Claims

1. Composition containing N-((3(5)-methyl-1H-pyrazol-1-yl)methyl)acetamide (MPA) (I) as nitrification inhibitor, N-(2-nitrophenyl)phosphoric acid triamide (2NPT) (II) as urease inhibitor, vegetable oil, at least one wax and optionally a diluent

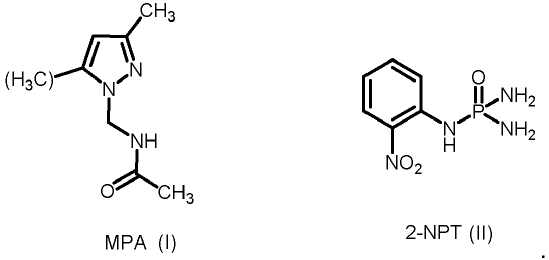

2. Composition according to claim 1, **characterized in that** N-((3(5)-methyl-1H-pyrazol-1-yl)methyl)acetamide and N-(2-nitrophenyl)phosphoric acid triamide (2NPT) are contained in a concentration of 10-40 wt%, based on the total composition.

3. Composition according to claim 1 or claim 2, **characterized in that** the vegetable oil is contained in a concentration of 30-85 wt.%, based on the total composition.

4. Composition according to any one of claims 1-3, **characterized in that** a wax is contained in an amount of 0.1 to 5 wt%, based on the total composition.

5. Composition according to any one of claims 1-4, **characterized in that** a diluent is contained in a concentration of 0.1 to 10 wt%, based on the total composition.

6. Composition according to any one of claims 1-5, **characterised in that** the diluent is selected from an N-substituted lactam, an ester, esterified abietic acid, isopropylidene glycerol, glycerol formal or mixtures thereof.

7. Composition according to claim 6, **characterized in that** the N-substituted lactam is selected from N-methylpyrrolidone and/or the ester is selected from a fatty acid alkyl ester.

8. Use of the composition according to any one of claims 1-7 for the preparation of storable finished fertilizers with dual nitrogen stabilization.

9. Process for the preparation of storable finished fertilizers with dual nitrogen stabilization, **characterized in that** granulated urea is treated with the composition according to any one of claims 1-7.

10. Storable finished fertilizer with dual nitrogen stabilization, containing the composition according to any one of claims 1-7.

11. Storable finished fertilizer according to claim 10, **characterized in that** the finished fertilizer contains 0.1 to 0.60 wt% of the composition according to any one of claims 1-7.

## Revendications

1. Composition contenant du N-((3(5)-méthyl-1H-pyrazol-1-yl)méthyl)acétamide (MPA) (I) en tant qu'inhibiteur de nitrification, du N-(2-nitrophényl)triamide d'acide phosphorique (2NPT) (II) en tant qu'inhibiteur d'uréase, de l'huile végétale, au moins une cire et éventuellement un diluant

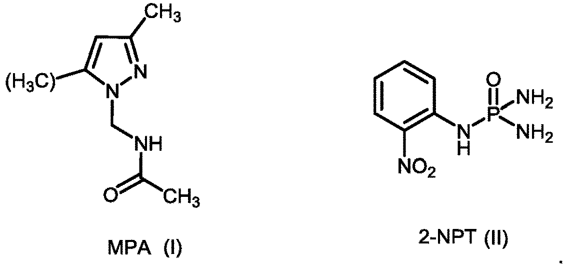

2. Composition selon la revendication 1, **caractérisée en ce que** le N-((3(5)-méthyl-1H-pyrazol-1-yl)méthyl)acétamide et le N-(2-nitrophényl)triamide d'acide phosphorique (2NPT) sont contenus en une concentration comprise entre 10 et 40 % en poids par rapport à la composition totale.

3. Composition selon la revendication 1 ou la revendication 2, **caractérisée en ce que** l'huile végétale est contenue en une concentration comprise entre 30 et 85 % en poids par rapport à la composition totale.

4. Composition selon l'une quelconque des revendications 1 à 3, **caractérisée en ce qu'**une cire est contenue en une quantité comprise entre 0,1 et 5 % en poids par rapport à la composition totale.

5. Composition selon l'une quelconque des revendications 1 à 4, **caractérisée en ce qu'**un diluant est contenu en une concentration comprise entre 0,1 et 10 % en poids par rapport à la composition totale.

6. Composition selon l'une quelconque des revendications 1 à 5, **caractérisée en ce que** le diluant est choisi parmi un lactame N-substitué, un ester, de l'acide abiétique estérifié, du glycérol d'isopropylidène, du glycérol formai ou des mélanges de ceux-ci.

7. Composition selon la revendication 6, **caractérisée en ce que** le lactame N-substitué est choisi parmi de la N-méthylpyrrolidone et/ou l'ester est choisi parmi un ester alkylique d'acide gras.

8. Utilisation de la composition selon l'une quelconque des revendications 1 à 7 pour la préparation d'engrais prêts à l'emploi stockables avec double stabilisation de l'azote.

9. Procédé de préparation d'engrais prêts à l'emploi stockables avec double stabilisation de l'azote, **caractérisé en ce que** de l'urée granulée est traitée avec la composition selon l'une quelconque des revendications 1 à 7.

10. Engrais prêt à l'emploi stockable avec double stabilisation de l'azote, contenant la composition selon l'une quelconque des revendications 1 à 7.

11. Engrais prêt à l'emploi stockable selon la revendication 10, **caractérisé en ce que** l'engrais prêt à l'emploi contient entre 0,1 et 0,60 % en poids de la composition selon l'une quelconque des revendications 1 à 7.
